Europäisches Patentamt

European Patent Office

Office européen des brevets

(19)

(11)  EP 1 030 677 B1

(12)  **FASCICULE DE BREVET EUROPEEN**

(45) Date de publication et mention
de la délivrance du brevet:
**06.02.2002   Bulletin 2002/06**

(51) Int Cl.[7]: **A61K 35/60**, A61K 31/715,
A61P 19/00

(21) Numéro de dépôt: **98954551.2**

(22) Date de dépôt: **10.11.1998**

(86) Numéro de dépôt international:
**PCT/FR98/02398**

(87) Numéro de publication internationale:
**WO 99/24046 (20.05.1999 Gazette 1999/20)**

(54) **COMPOSITION ORALE SYNERGIQUE ASSOCIANT DE L'ADN DE SAUMON A DE L'HYDROXYPROLINE**

SYNERGISTISCHE ORALE ZUSAMMENSETZUNGEN, DIE EINE KOMBINATION VON LACHS-DNA MIT HYDROXYPROLIN ENTHALTEN

SYNERGETIC ORAL COMPOSITION COMBINING SALMON DNA WITH HYDROXYPROLINE

(84) Etats contractants désignés:
**AT BE CH CY DE DK ES FI FR GB GR IE IT LI LU MC NL PT SE**

(30) Priorité:  **10.11.1997  FR 9714093**

(43) Date de publication de la demande:
**30.08.2000   Bulletin 2000/35**

(73) Titulaire: **Pierre Fabre Dermo-Cosmetique**
**92100 Boulogne (FR)**

(72) Inventeurs:
• **COUSSE, Henri**
**F-31860 Pins Justaret (FR)**
• **POTHERAT, Jean-Jacques**
**F-81440 Lautrec (FR)**
• **ROUANET, Max**
**F-66360 Olette (FR)**

(74) Mandataire: **Ahner, Francis et al**
**Cabinet Régimbeau 20, rue de Chazelles**
**75847 Paris cedex 17 (FR)**

(56) Documents cités:
**EP-A- 0 704 216          WO-A-94/22453**
**FR-A- 2 632 186          US-A- 4 473 551**

• **DATABASE WPI Section Ch, Week 9314 Derwent Publications Ltd., London, GB; Class B04, AN 93-112771 XP002074744 & JP 05 051400 A (KANEKA CORP)**

**Description**

La présente invention concerne de nouvelles compositions orales synergiques contenant de l'hydroxyproline associées à de l'ADN de saumon. Les compositions peuvent en outre comprendre de l'acide chondroïtine sulfurique.

De manière habituelle, dans les formulations de l'état antérieur de la technique, l'hydroxyproline était apportée par des protéines de mammifères, en particulier par des gélatines hydrolysées.

Les zoonoses transmissibles ont jeté un discrédit important sur les produits d'opochimiothérapie et, en plus des exigences réglementaires (scores BGA, projet de réglementation européen concernant les extraits d'organes ovin, bovin et caprin) s'ajoutent les pressions des médias et la phobie des consommateurs.

Pour palier ces inconvénients, dans les formulations relatives à la présente invention, l'hydroxyproline a été apportée par des produits d'origine marine, et l'acide chondroïtine sulfurique par des produits d'origine marine, végétale ou aviaire, l'ADN est obtenu à partir de laitances plus particulièrement de saumon.

La présente invention vise des compositions orales synergiques associant de l'hydroxyproline (HP) d'origine marine à de l'ADN de saumon et éventuellement, à de l'acide chondroïtine sulfurique (ACS) d'origine marine, végétale ou aviaire.

Selon une caractéristique particulière, les rapports pondéraux sont les suivants :

$$1 \leq HP/ACS \leq 15$$

$$5 \leq HP/ADN \leq 20$$

et de préférence :

$$5 \leq HP/ACS \leq 10$$

$$12 \leq HP/ADN \leq 18$$

Alors que, habituellement l'acide chondroïtine sulfurique était obtenu à partir de trachées de bovins, l'ACS utilisé dans le cadre de la présente invention se présente avantageusement sous forme d'extraits d'holothuries (concombre de mer), de peaux de seiche, de poissons cartilagineux tels que de la raie ou de la roussette, ou encore de bréchets aviaires, en particulier, de poulets et de dinde.

L'hydroxyproline utilisée dans les formulations orales, objet de la présente invention, se présente sous la forme d'hydrolysats de poissons (en particulier, de cabillaud, de thon ou de morue).

L'ADN entrant dans les compositions selon l'invention est présent sous la forme de laitance de saumon titrée en ADN ou bien d'ADN purifiée.

Conformément à une autre caractéristique de la présente invention, les compositions peuvent en plus contenir une fraction insaponifiable de maïs.

L'introduction d'une telle fraction insaponifiable de maïs est particulièrement avantageuse lorsque l'ACS est associé à des protéines de mais riches en hydroxyproline.

Les compositions orales indiquées ci-après, à titre d'exemple, sont destinées à mieux illustrer l'objet de la présente invention, sans pour autant en limiter la portée.

| EXEMPLE 1 : pour une dose de 12 g | | |
|---|---|---|
| | | Plus précisément |
| Hydrolysat de collagène | | |
| Riche en HP | 5 à 8 g | 7,2 g |
| Fructose | 2,5 à 4,5 g | 3, 86 g |
| Cystine | 0,04 à 0,10 | 0,06 g |
| Acide citrique | 0,2 à 0,5 g | 0,36 |
| Composition aromatique | | |
| Orange/banane | 0,2 à 1 g | 0,42 g |

(suite)

| EXEMPLE 1 : pour une dose de 12 g | | |
|---|---|---|
| | Plus précisément | |
| Hydrolysat de collagène | | |
| ADN de saumon | 0,05 à 0,5 g | 0,05 g |

Cette formulation apporte 0,7 g d'hydroxyproline et 0,05 g d'ADN par unité de prise.

| Exemple 2 : complément alimentaire nutrithérapique | |
|---|---|
| Hydroxyproline d'hydrolysat de collagène marin | 6 g |
| Laitance de saumon titrée en ADN, ou ADN purifié | 0,05 g |
| Acide citrique | 0,5 g |

Les compositions orales synergiques, objet de la présente invention, sont utiles comme complément alimentaire en nutrithérapie pour éviter les troubles liés au vieillissement du cartilage et retarder la dégénérescence de ce dernier.

## ETUDE D'EFFICACITE

a) Protocole

L'étude a été effectuée avec 80 personnes randomisées en 2 groupes. Il s'agit de patients souffrant de douleurs arthrosiques qui consomment en général des antiinflammatoires non stéroidiens (AINS) (aspirine, ibuprofène, etc) dont l'âge varie de 37 à 85 ans.

Il s'agit de 52 femmes et de 28 hommes.

Le premier groupe de 40 patients (14 hommes et 26 femmes) a reçu pendant 60 jours une préparation selon l'exemple 1 sans ADN, poudre absorbée en suspension dans l'eau matin et soir.

Le deuxième groupe de 40 patients (14 hommes et 26 femmes) a reçu pendant 60 jours la préparation complète selon l'exemple 1 matin et soir.

Les résultats ont été évalués en fonction du nombre d'épisodes douloureux évalués par les patients, de la consommation AINS, nombre de prise au cours des 60 jours et la souplesse articulaire flexion dans les cas de gonarthrose et coxarthrose.

Au début de l'étude, les volontaires ont reçu 120 sachets dose et un questionnaire, en cours de traitement un contact a été établi pour vérifier l'observance (prise des sachets) et les fiches d'autocontrôle.

Nous avons pu recueillir 75 fiches dont 73 ont été exploitables (5 patients ne sont pas revenus à la date fixée en fin de traitement).

b) Les résultats analysés sont reportés dans le tableau ci-dessous :

| EVALUATION | GROUPE 1 SANS ADN | GROUPE 2 AVEC ADN Exemple 1 |
|---|---|---|
| Consommation AINS (nombre de prises) | 20 $\pm$ 6 | 14 $\pm$ 7 |
| Nombre d'épisodes douloureux cochés sur fiches | 62 | 41 |
| Amélioration de la souplesse articulaire en fin de traitement contact lors de la remise des fiches | + 15 % | + 30 % |

3)Discussion

Malgré le faible nombre de cas pour une étude en ouvert sans placebo, la différence de résultats est surprenante, l'ADN ayant un effet potentialisateur inattendu.

Cette potentialisation pourrait s'expliquer par une amélioration du passage et une protection de l'hydroxyproline en présence d'ADN. Un effet similaire a été constaté avec l'ACS qui possède des propriétés particulières mises en évidence avec le Ca qui est capté et dont l'absorption est améliorée en présence de l'ACS.

Par contre, c'est la première fois qu'un effet de potentialisation a été observé avec l'hydroxyproline associée à l'ACS ou à l'ADN.

Les compositions orales, objet de la présente invention, ne sont pas seulement utiles comme complément alimentaire utile en nutrithérapie, mais elles trouvent également leur application comme compositions cosmétologiques administrables par voie orale. Dans ce cas, elles sont utiles comme protecteur du photovieillissement et/ou comme régénérateur du collagène de la peau.

**Revendications**

1. Complément alimentaire comprenant une association synergique d'hydroxyproline (HP) d'origine marine, et d'ADN de saumon.

2. Complément alimentaire selon la revendication 1, **caractérisé en ce que** l'hydroxyproline est présente sous la forme d'un hydrolysat de poisson, en particulier de cabillaud, de thon ou de morue.

3. Complément alimentaire selon l'une des revendications 1 et 2, **caractérisé en ce que** l'ADN est présent à partir de laitance de saumon titrée en ADN ou d'ADN purifié.

4. Complément alimentaire selon l'une des revendications 1 à 3, **caractérisé par** le rapport pondéral :

$$5 \leq HP / ADN \leq 20,$$

et de préférence

$$12 \leq HP / ADN \leq 18.$$

5. Complément alimentaire selon l'une des revendications 1 à 4, **caractérisé en ce qu'**il comprend en outre de l'acide chondroïtine sulfurique (ACS) d'origine marine, végétale ou aviaire.

6. Complément alimentaire selon la revendication 5, **caractérisé par** le rapport pondéral :

$$1 \leq HP / ACS \leq 15,$$

et de préférence

$$5 \leq HP / ACS \leq 10.$$

7. Complément alimentaire selon l'une des revendications 5 et 6, **caractérisé en ce que** l'acide chondroïtine sulfurique est présent sous la forme d'extrait d'holothuries, de peau de seiche, de poissons cartilagineux tels que la raie et la roussette ou encore de bréchets aviaires, en particulier de poulets ou de dindes.

8. Complément alimentaire selon l'une des revendications 1 à 7, **caractérisé en ce qu'**il contient en plus une fraction insaponifiable, en particulier, de maïs.

9. Complément alimentaire selon l'une des revendications 1 à 4, **caractérisé en ce qu'**il répond à la formulation suivante :

| Hydrolysat de collagène marin | 5 à 8 g |
|---|---|
| Fructose | 2,5 à 4,5 g |
| Cystine | 0,04 à 0,10 g |

(suite)

| Acide citrique | 0,2 à 0,5 g |
| Composition aromatique | |
| Orange / banane | 0,2 à 1 g |
| ADN de saumon | 0,05 à 0,5 g |

**10.** Complément alimentaire selon l'une des revendications 1 à 4, **caractérisé en ce qu'**il répond à la formulation suivante :

| Hydroxyproline d'hydrolysat de collagène marin | 6 g |
| Laitance de saumon titrée en ADN ou ADN purifié | 0,05 g |
| Acide citrique | 0,5 g |

**11.** Application des compléments alimentaires selon l'une des revendications 1 à 10 en cosmétologie, comme protecteur du photovieillissement et/ou régénérateur du collagène de la peau.

**Patentansprüche**

**1.** Nahrungsmittelergänzung, umfassend eine synergistische Verbindung von Hydroxyprolin (HP) aus Meeresursprung und Lachs-DNS.

**2.** Nahrungsmittelergänzung nach Anspruch 1, **dadurch gekennzeichnet, dass** das Hydroxyprolin in Form eines Hydrolysats von Fisch, insbesondere von Schellfisch, Thunfisch oder Kabeljau, vorliegt.

**3.** Nahrungsmittelergänzung nach einem der Ansprüche 1 und 2, **dadurch gekennzeichnet, dass** die DNS ausgehend von Lachsmilch, die bezüglich DNS titriert ist, oder von gereinigter DNS vorliegt.

**4.** Nahrungsmittelergänzung nach einem der Ansprüche 1 bis 3, **gekennzeichnet durch** das Gewichtsverhältnis:

$$5 \leq HP/DNS \leq 20$$

und vorzugsweise

$$12 \leq HP/DNS \leq 18.$$

**5.** Nahrungsmittelergänzung nach einem der Ansprüche 1 bis 4, **dadurch gekennzeichnet, dass** sie darüber hinaus Chondroitinschwefelsäure (CSS) aus Meeres-, pflanzlichem oder Vogelursprung umfasst.

**6.** Nahrungsmittelergänzung nach Anspruch 5, **gekennzeichnet durch** das Gewichtsverhältnis:

$$1 \leq HP/CSS \leq 15$$

und vorzugsweise

$$5 \leq HP/CSS \leq 10.$$

**7.** Nahrungsmittelergänzung nach einem der Ansprüche 5 und 6, **dadurch gekennzeichnet, dass** die Chondroitinschwefelsäure in Form eines Extraktes von Seegurken, von Tintenfischhaut, von Knorpelfischen, wie dem Rochen und dem Katzenhai, oder auch von Vogelbrustbeinkämmen, insbesondere von Hühnchen oder Truthähnen, vorliegt.

8. Nahrungsmittelergänzung nach einem der Ansprüche 1 bis 7, **dadurch gekennzeichnet, dass** sie weiter eine unverseifbare Fraktion, insbesondere von Mais, enthält.

9. Nahrungsmittelergänzung nach einem der Ansprüche 1 bis 4, **dadurch gekennzeichnet, dass** sie der folgenden Formulierung entspricht:

| | |
|---|---|
| Hydrolysat von Meeres-Kollagen | 5 bis 8 g |
| Fructose | 2,5 bis 4,5 g |
| Cystin | 0,04 bis 0,10 g |
| Citronensäure | 0,2 bis 0,5 g |
| Aromazusammensetzung | |
| Orange/Banane | 0,2 bis 1 g |
| Lachs-DNS | 0,05 bis 0,5 g |

10. Nahrungsmittelergänzung nach einem der Ansprüche 1 bis 4, **dadurch gekennzeichnet, dass** sie der folgenden Formulierung entspricht:

| | |
|---|---|
| Hydroxyprolin aus einem Hydrolysat von Meeres-Kollagen | 6 g |
| Lachsmilch, bezüglich DNS titriert, oder gereinigte DNS | 0,05 g |
| Citronensäure | 0,5 g |

11. Anwendung der Nahrungsmittelergänzungen nach irgendeinem der Ansprüche 1 bis 10 in der Kosmetik als Schutz vor Lichtalterung und/oder Regenerationsmittel des Kollagens der Haut.

**Claims**

1. Dietary supplement comprising a synergistic combination of hydroxyproline (HP) of marine origin, and salmon DNA.

2. Dietary supplement according to Claim 1, **characterized in that** the hydroxyproline is present in the form of a fish, in particular codfish, tuna or cod, hydrolysate.

3. Dietary supplement according to either of Claims 1 and 2, **characterized in that** the DNA is present from salmon soft roe having a DNA content or from purified DNA.

4. Dietary supplement according to one of Claims 1 to 3, **characterized by** the weight ratio:

$$5 \leq HP / DNA < 20,$$

and preferably

$$12 \leq HP / DNA < 18.$$

5. Dietary supplement according to one of Claims 1 to 4, **characterized in that** it comprises, in addition, chondroitin sulphuric acid (CSA) of marine, plant or avian origin.

6. Dietary supplement according to Claim 5, **characterized by** the weight ratio:

$$1 \leq HP / CSA \leq 15,$$

and preferably

$$5 \leq HP / CSA \leq 10.$$

7. Dietary supplement according to either of Claims 5 and 6, **characterized in that** the chondroitin sulphuric acid is present in the form of an extract of holothurians, of cuttlefish skin, of cartilaginous fish such as ray and dogfish or of avian, in particular chicken or turkey, carinas.

8. Dietary supplement according to one of Claims 1 to 7, **characterized in that** it contains, in addition, an unsaponifiable fraction, in particular, of maize.

9. Dietary supplement according to one of Claims 1 to 4, **characterized in that** it corresponds to the following formulation:

| | |
|---|---|
| marine collagen hydrolysate | 5 to 8 g |
| fructose | 2.5 to 4.5 g |
| cystine | 0.04 to 0.10 g |
| citric acid | 0.2 to 0.5 g |
| orange/banana aromatic composition | 0.2 to 1 g |
| salmon DNA | 0.05 to 0.5 g |

10. Dietary supplement according to one of Claims 1 to 4, **characterized in that** it corresponds to the following formulation:

| | |
|---|---|
| hydroxyproline of marine collagen hydrolysate | 6 g |
| salmon soft roe with a content of DNA or purified DNA | 0.05 g |
| citric acid | 0.5 g |

11. Application of the dietary supplements according to one of Claims 1 to 10 in cosmetology, as protectant from photoageing and/or skin collagen regenerator.